# EUROPEAN PATENT SPECIFICATION

(11) **EP 3 711 779 B1**
(45) Date of publication and mention of the grant of the patent: **06.09.2023**
(21) Application number: 18877526.6
(22) Date of filing: 13.11.2018
(51) Int. Cl.: A61K 31/575, A61K 31/7048, A61K 31/585, A61K 31/4375, A61K 35/644, A61K 31/20, A61K 31/201, A61K 31/202, A61K 36/539, A61K 36/756, A61K 36/718, A61K 47/28, A61K 47/44, A61K 36/66, A61K 35/62, A61K 31/4741, A61K 31/198, A61K 31/195, A61P 17/02, A61P 9/00, A61P 1/16, A61P 11/00, A61P 13/12, A61P 9/10, A61P 39/06, A61K 31/366, A61P 43/00

(54) **APPLICATION OF PHARMACEUTICAL COMPOSITION IN REGULATION OF FIBROBLAST GROWTH**
ANWENDUNG EINER PHARMAZEUTISCHEN ZUSAMMENSETZUNG ZUR REGULIERUNG DES FIBROBLASTENWACHSTUMS
APPLICATION D'UNE COMPOSITION PHARMACEUTIQUE DANS LA RÉGULATION DE LA CROISSANCE DES FIBROBLASTES

(30) Priority: 14.11.2017 CN 201711119842
(43) Date of publication of application: 23.09.2020
(73) Proprietor: Li, Li, Arcadia, California 91007 (US); Beijing Rongxiang Institute Of Regenerative Medicine Co., Ltd., Beijing 100176 (CN)
(72) Inventor: LI, Li, Arcadia, CA 91007 (US)
(74) Representative: Pistolesi, Roberto
(86) International application number: PCT/CN2018/115174
(87) International publication number: WO 2019/096114

(56) References cited:
- WO-A1-03/037360
- WO-A2-03/001982
- CN-A- 1 145 779
- CN-A- 1 404 835
- CN-A- 107 899 015
- KI-SUK KIM ET AL: "Effects of beta-sitosterol derived from Artemisia capillaris on the activated human hepatic stellate cells and dimethylnitrosamine-induced mouse liver fibrosis", BMC COMPLEMENTARY AND ALTERNATIVE MEDICINE, BIOMED CENTRAL LTD., LONDON, GB, vol. 14, no. 1, 27 September 2014 (2014-09-27), page 363, XP021199401, ISSN: 1472-6882, DOI: 10.1186/1472-6882-14-363
- ATIYEH BISHARA S ET AL: "Improving Scar Quality: A Prospective Clinical Study", AESTHETIC PLASTIC SURGERY, SPRINGER VERLAG, NEW YORK, NY, US, vol. 26, no. 6, 1 November 2002 (2002-11-01), pages 470-476, XP036370692, ISSN: 0364-216X, DOI: 10.1007/S00266-002-2019-5 [retrieved on 2002-11-01]

## Description

### FIELD OF THE INVENTION

The present invention relates to a use of a pharmaceutical composition for regulating fibroblast growth.

### BACKGROUND OF THE INVENTION

Chinese patent ZL 02105541.6 discloses a pharmaceutical composition suitable for oral administration, comprising a homogenous mixture of edible oil, beeswax and β-sitosterol, wherein the beeswax in the composition forms microcrystals, the content of the beeswax is 0.5 to 50% and the content of the β-sitosterol is at least 0.1% by weight based on the total weight of the composition. In addition, the composition may also comprise other pharmaceutical ingredients, and is used to deliver other active ingredients to the gastrointestinal tract for treating various diseases.

Moreover, this pharmaceutical composition is mainly used to protect mucosal tissues from damage caused by irritants, and to promote the repair and regeneration of damaged or dysfunctional gastrointestinal mucosal tissues. It is particularly used for the treatment of gastrointestinal disorders such as gastritis, peptic ulcer, reflux esophagitis, dyspepsia and gastric cancer, as well as for the reconstruction of the physiological structure and function of mucosal tissues.

WO 03/001982 discloses a composition comprising edible oil, β-sitosterol and beeswax for use in the treatment of tissue disorders caused by aging and for preventing scars formation. The composition decreases fibroblast grotwh.

In the present application, "pharmaceutical composition", "pharmaceutical composition according to the present invention" or "the present pharmaceutical composition" refers to a pharmaceutical composition as defined in the claims comprising a homogenous mixture of edible oil, beeswax and β-sitosterol, wherein the beeswax in the composition forms microcrystals, the content of the beeswax is 0.5 to 50% and the content of the β-sitosterol is 0.1 to 20% by weight based on the total weight of the composition.

Fibroblasts are the main cells in the dermis of the skin, which are called fibrocytes at resting state. They are a kind of low-metabolism, inactive cells under normal condition. These "hibernated" cells are sparsely distributed in connective tissue, and the density of the cell is high merely in the soft tissue around the blood vessels. Fibroblasts have obvious functional diversity, and are key cells for organ and tissue fibrosis, scar formation and tissue aging.

### About organ and tissue fibrosis

The pathogenesis of organ and tissue fibrosis has evolved from the changes of histopathology in the past to the research of cell and molecular biology at present. Although the specific pathogenesises of fibrosis in different organs and tissues are somewhat different, they have many commonalities. Fibrosis is a slow, dynamic and complex process, involving the interaction and inter-adjustment of various factors and links such as cells, cytokines and extracellular matrix (ECM). The final outcome is the deposition of a large amount of ECM^{[1]}. Organ and tissue fibrosis starts from the substantial cell damage caused by various reasons (inflammation, immunity, poison, ischemia, hemodynamic changes and the like), followed by inflammatory degeneration and necrosis of cells, which activates the corresponding macrophages (pre-inflammatory cells) to release various active factors such as cytokines and growth factors, the latter activates resting extracellular matrix producing cells (ECM-producing cells), which then transform into myofibroblasts (MFB). Myofibroblasts proliferate and secrete cytokines, and then act on macrophages by paracrine. Myofibroblasts synthesize large amounts of ECM components such as collagen, while ECM degradation is reduced, resulting in organ and tissue fibrosis. In such pathogenesis, the conversion of ECM-producing cells into myofibroblasts is a core event and a necessary step for organ and tissue fibrosis and scar formation, wherein it is determined that the cell type of ECM-producing cells is mainly fibroblast, and there are also other types of cells involved. Their role in the pathogenesis of fibrosis has been the focus of research in recent years. Normal fibroblast is at resting state, its metabolism and function are not active. However, under pathological conditions, fibroblast not only changes in morphology, but also shows obvious functional changes such as proliferation, secretion of cytokines, synthesis of large amounts of ECM, and production of protein degrading enzymes. The series of changes is called activation. The activation of ECM-producing cell is a key step and core link in the formation of fibrosis.

Studies on the activation of fibroblasts indicate that under normal conditions, they are a kind of non-activated cells with low metabolism. When the fibroblast is stimulated, it is transformed from proliferative cell into cell that proliferates and overproduces matrix, i.e. fibroblast activation occurs. The activated fibroblast undergoes functional and phenotypic changes and is transformed into myofibroblast that expresses α-smooth muscle actin (α-SMA), the ability of the latter to synthesize ECM is significantly improved. It is thought that fibroblast activation is a key step during the formation of fibrosis, factors such as growth factors and cytokines by autocrine and paracrine, direct cell-to-cell contact, the effect of ECM via integrin, and local tissue hypoxia may stimulate fibroblast activation.

Myofibroblasts transformed from fibroblasts are a group of smooth muscle cell-like cells^{[2]}, which have α-SMA in the cytoplasm. The myofibroblast has similar functions. The nuclear membrane is wrinkled, and the cytoplasm contains a large number of bundled filaments, actin, and expanded rough endoplasmic reticulum. The myofibroblast mainly expresses α-SMA, which is a marked antigen of active myofibroblast. The myofibroblast plays a biological role by secreting cytokines, chemokines, growth factors, ECM and proteases. The myofibroblast and fibroblast are very closely related. In summary, the activated fibroblast can undergo phenotypic changes and transform into myofibroblast; the myofibroblast has both the function and structural characteristics of fibroblast and smooth muscle cell, and can express α-SMA; the expression of α-SMA by fibroblast is a sign of its activation and damage.

A large number of studies have demonstrated that the pathogenesis of organ and tissue fibrosis is extremely complicated, involving the proliferation and activation of ECM-producing cell, the generation, release and regulation of various growth factors, cytokines and vasoactive substances, as well as the balance between ECM synthesis and degradation and the like. Therefore, the occurrence and development of organ fibrosis is a very complicated process involving the interaction and regulation of multiple factors such as cells, cytokines and ECM. Since the pathogenesis of fibrosis is extremely complicated and involves many factors, there should be more than one target for treatment. There are currently three most important targets for the treatment of fibrosis: ECM-producing cell, cytokine and ECM, and the most important of which is ECM-producing cell.

Recent studies have demonstrated that inhibiting the proliferation and activation of ECM-producing cell is the central strategy for anti-fibrosis. For example, it was found that γ-interferon, retinoic acids, cAMP inducers, endothelin receptor antagonists and certain antioxidants can inhibit the activation of hepatic stellate cell (an ECM-producing cell). However, these are mostly laboratory research results, which are still far from clinical application. Inducing the apoptosis of activated ECM-producing cell is an important target for anti-fibrosis. However, the method of selectively promoting the apoptosis of ECM-producing cell is not yet ideal at present.

### About scar formation

Fibroblasts are the main cells that synthesize collagen in the body, and are closely related to scar formation. Among the various types of collagen synthesized by fibroblast, the type I and type III collagen are most closely related to scar formation. Various types of scar fibroblasts all have the characteristic of increased collagen synthesis, since the activity of collagen synthase is significantly enhanced. For example, the activity of collagen synthase proline hydroxylase in keloid is significantly higher than that in hypertrophic scar, and its collagen synthesis is 20 times that of normal skin and 3 times that of hypertrophic scar.

It is reported of using silver staining nucleolar tissue area method to determine the activity and proliferation of fibroblast. It was found that the cultured normal skin fibroblast, hypertrophic scar fibroblast and keloid fibroblast have significant differences in activity and proliferation, and the latter being the strongest. The proliferation activity of fibroblast is also different in different tissues. The test of proliferating cell nuclear antigen (PCNA) found that the PCNA positive rate of fibroblasts in normal skin was 38.8% ± 6.3%, while that of hypertrophic scar was 45.6% ± 10.6%, and that of keloid was as high as 75.1% ± 8.1 %.

The keloid fibroblasts, hypertrophic scar fibroblasts and normal skin fibroblasts were cultured to observe the relationship between fibroblast and scar formation. The expression of PCNA, p16 protein (which can induce the cell to stop at G₁ phase), type I collagen, type III collagen and corresponding procollagen genes were determined before and after cell contact. The proliferation and biosynthetic function of various fibroblasts were studied. The results showed that when the cells did not contact with each other, the keloid fibroblast, hypertrophic scar fibroblast and normal skin fibroblast all expressed PCNA, and hardly expressed p16. Once the fibroblasts contacted with each other, the normal skin fibroblast expressed less PCNA obviously or did not express PCNA, while p16 expression increased, and the cells appeared as a monolayer with directional arrangement, indicating that the normal skin fibroblast had contact inhibition and density inhibition. The keloid fibroblast still expressed high PCNA, while p16 expression was insignificant, and the cells appeared as a disordered arrangement with overlapping and stratification, indicating that the keloid fibroblast did not have contact inhibition and density inhibition. The hyperplastic scar fibroblast was in between. After cell contact, the synthesis of type I collagen and type III collagen in normal skin fibroblasts decreased significantly, accompanied by a significant decrease in the expression of corresponding procollagen genes. However after cell contact, the biosynthesis in keloid fibroblast and hypertrophic scar fibroblast was still vigorous, and type I and III collagen genes were still strongly expressed.

Many scholars have conducted in-depth studies on the growth and proliferation characteristics of scar tissue fibroblasts in vitro using cell culture techniques. The characteristics between normal skin fibroblast and keloid fibroblast were compared using this technique. It was found that there was no obvious difference in their morphological size and their growth kinetics. However, the division of keloid fibroblast may require less stimulation of growth factor.

The relationship between growth factor and scar is also very important. It is known at present that transforming growth factor β1 (TGFβ₁) is a very important growth factor. Connective tissue growth factor (CTGF) is a polypeptide with platelet-derived growth factor (PDGF)-like activity, which is synthesized by skin fibroblasts after being activated by TGF. When skin fibrosis occurs, CTGF is positive. Abnormal expression of CTGF gene will cause skin sclerosis and fibrosis. In addition, TGFβ₁ is currently the only CTGF inducer confirmed. This also illustrates the role of TGF in fibrous pathology.

The understanding on the regulation of cell cycle provides a theoretical basis for specifically controlling the wound healing process. People now hope to control the pathological fibrosis process of scar by regulating the growth and proliferation of fibroblast.

The biggest physiological significance of fibroblast is tissue repair^{[3-5]}. The normal function of fibroblast is one of the key factors for the successful completion of tissue repair. It is known that fibroblast is the effector cell of scar formation, and the abnormal proliferation of fibroblast is the main reason for the excessive proliferation and persistent existence of pathological scar. The current point of view has focused on the basic point of the imbalance of fibroblast proliferation/apoptosis (apoptosis refers to the natural death of the cell, which is initiated by the internal mechanism of the cell and is very important for maintaining physiological balance). It is demonstrated that the pathological healing of skin damage is due to the excessive proliferation of fibroblast. During the remodeling phase of skin repair, the proliferation and apoptosis of fibroblast present in the bud tissue are dysregulated. The proliferation activity of fibroblast is significantly higher than that of normal skin fibroblast, while the apoptosis of fibroblast lags behind the cell proliferation. This causes the accumulation of fibroblasts during the tissue repair process, resulting in the synthesis of more type I collagen, an increased ratio of type I/III collagen (opposite to the phenomenon of decreased ratio of type I/III collagen during wound healing process) and a pathological scar healing. Effective regulation of the balance between proliferation/apoptosis of fibroblast is the key to inhibit the formation of pathological scar.

### SUMMARY OF THE INVENTION

The technical problem to be solved by the present invention is to regulate fibroblast growth and inhibit organ fibrosis by using the above known pharmaceutical composition.

In an aspect, the present disclosure provides a pharmaceutical composition for regulating fibroblast growth, wherein the pharmaceutical composition is a pharmaceutical composition suitable for oral administration comprising a homogenous mixture of edible oil, beeswax and β-sitosterol, wherein the beeswax in the composition forms microcrystals, the content of the beeswax is 0.5 to 50% and the content of the β-sitosterol is 0.1% to 20% by weight based on the total weight of the composition.

The "regulating fibroblast growth" refers to inhibiting fibroblast growth and reproduction and inactivating fibroblast.

The present invention provides a pharmaceutical composition for use as a medicament for treating or preventing organ fibrosis, scar formation and/or tissue aging, wherein the pharmaceutical composition is a pharmaceutical composition suitable for oral administration as defined in claim 1 comprising a homogenous mixture of edible oil, beeswax and β-sitosterol, wherein the beeswax in the composition forms microcrystals, the content of the beeswax is 0.5 to 50% and the content of the β-sitosterol is 0.1% to 20% by weight based on the total weight of the composition.

In a specific embodiment, the organ comprises heart, liver, lungs, kidneys and bone marrow.

In a specific embodiment, the organ is an organ from a mammal, and the mammal is preferably a human.

In a specific embodiment, the content of the β-sitosterol in the pharmaceutical composition is 0.5 to 20% by weight.

In a specific embodiment, the content of the β-sitosterol in the pharmaceutical composition is 1 to 10% by weight.

In a specific embodiment, the content of the beeswax in the pharmaceutical composition is 3 to 30% by weight.

In a specific embodiment, the content of the beeswax in the pharmaceutical composition is 5 to 20% by weight.

In a specific embodiment, the content of the beeswax in the pharmaceutical composition is 6 to 10% by weight.

In a specific embodiment, the edible oil in the pharmaceutical composition is corn oil, wheat germ oil, soybean oil, rice bran oil, rapeseed oil, sesame oil or fish oil.

In a specific embodiment, the pharmaceutical composition further comprises propolis, and the content thereof is 0.1 to 30% by weight.

In a specific embodiment, the pharmaceutical composition comprises water, and the content thereof is less than or equal to 1% by weight.

In a specific embodiment, the dosage form of the oral pharmaceutical composition is selected from the group consisting of tablet, pill, capsule, emulsion, gel, syrup and suspension.

In a specific embodiment, the pharmaceutical composition further comprises *Scutellaria baicalensis* or the extract of *Scutellaria baicalensis,* and the content of *Scutellaria baicalensis* or the extract of *Scutellaria baicalensis* containing 0.1 to 0.5% of baicalin is 2 to 5% by weight based on the total weight of the composition.

The extract of *Scutellaria baicalensis* is an extract of *Scutellaria baicalensis* with water, organic solvent such as oil and ethanol, or a combination of water and organic solvent. More preferably, the extract is an extract of 1 to 50% by weight of *Scutellaria baicalensis* in an edible oil, preferably sesame oil. The radix of *Scutellaria baicalensis* is preferred. *Scutellaria baicalensis* is one or more *Labiatae* plants selected from the group consisting of *Scutellaria viscidula bunge, Scutellaria amoena, Scutellaria rehderiana Diels, Scutellaria ikonnikovii Juz, Scutellaria likiangensis* and *Scutellaria hypericifolia.*

In a specific embodiment, the pharmaceutical composition further comprises *Cortex Phellodendri* or the extract of *Cortex Phellodendri,* and the content of *Cortex Phellodendri* or the extract of *Cortex Phellodendri* containing 0.1 to 1% of obaculactone is 2 to 5% by weight based on the total weight of the composition.

The the extract of *Cortex Phellodendri* is an extract of *Cortex Phellodendri* with water, organic solvent such as oil and ethanol, or a combination of water and organic solvent. More preferably, the extract is an extract of 1 to 50% by weight of *Cortex Phellodendri* in an edible oil, preferably sesame oil. The cortex of *Cortex Phellodendri* is preferred. *Cortex Phellodendri* is one or more plants selected from the group consisting of *Phellodendron chinense Schneid, Phellodendron amurense, Phellodendron chinense Schneid var. omeiense, Phellodendron Schneid var. yunnanense* and *Phellodendron chinense Schneid var. falcutum.*

In a specific embodiment, the pharmaceutical composition further comprises 2 to 5% of *Coptis chinensis* or the extract of *Coptis chinensis* containing 0.1 to 1% of berberine by weight based on the total weight of the composition.

The extract of *Coptis chinensis* is an extract of *Coptis chinensis* with water, organic solvent such as oil and ethanol, or a combination of water and organic solvent. Preferably, the extract is an extract of 1 to 50% by weight of *Coptis chinensis* in an edible oil, preferably sesame oil. The radix of *Coptis chinensis* is preferred. *Coptis chinensis* is one or more *Ranunculaceae* plants selected from the group consisting of *Coptis deltoidea C. Y.Cheng et Hsial, Coptis omeiensis* and *Coptis teeta Wall.*

According to the present invention, the pharmaceutical composition further comprises 2 to 5% of *Scutellaria baicalensis* or the extract of *Scutellaria baicalensis* containing 0.1 to 0.5% of baicalin, 2 to 5% of *Cortex Phellodendri* or the extract of *Cortex Phellodendri* containing 0.1 to 1% of obaculactone, 2 to 5% of *Coptis chinensis* or the extract of *Coptis chinensis* containing 0.1 to 1% of berberine, 2 to 10% of *Pericarpium Papaveris* or the extract of *Pericarpium Papaveris* containing 0.1 to 1% of narcotoline, and 2 to 10% of earthworm or earthworm extract containing amino acid, by weight based on the total weight of the composition.

The extract of *Pericarpium Papaveris* is an extract of *Pericarpium Papaveris* with water, organic solvent such as oil and ethanol, or a combination of water and organic solvent. Preferably, the extract is an extract of 1 to 50% by weight of *Pericarpium Papaveris* in an edible oil, preferably sesame oil.

The earthworm extract is an extract of earthworm with water, organic solvent such as oil and ethanol, or a combination of water and organic solvent. More preferably, the extract is an extract of 1 to 50% by weight of earthworm in an edible oil.

The extraction of *Scutellaria baicalensis, Cortex Phellodendri, Coptis chinensis, Pericarpium Papaveris* and earthworm can be carried out according to the method described in Chinese Patent ZL 93100276.1 or Chinese Patent ZL 02105541.6.

In a specific embodiment, the pharmaceutical composition comprises 7% of beeswax, 1% of sterol, 0.5% of obaculactone, 0.3% of baicalin and 0.5% of berberine by weight based on the total weight of the composition.

In a specific embodiment, the beeswax has microcrystals with a length of 0.1 to 100 microns.

In a specific embodiment, at least two microcrystals of the beeswax in the pharmaceutical composition are polymerized into a microcrystal complex.

In a specific embodiment, the microcrystals of the beeswax are sufficiently uniformly dispersed in the edible oil.

It was determined in the skin graft of suckling mouse that the pharmaceutical composition of the present invention has a very significant inhibition effect on fibroblast. Fibroblasts growing out of the grafts were significantly inhibited by the pharmaceutical composition of the present invention, and changed in morphology. The cells turned black and shrinked until they died and dissolved. The effect of the pharmaceutical composition on fibroblasts that just grow out of the skin graft can also be observed under the microscope. In fact, the cells are inhibited at the early stage of growth without a chance of diffusion and growth. With respect to a large amount of fibroblasts growing in a short period of time, the cells will not be immediately inhibited or die due to the temporary balance of the pharmaceutical composition and fibroblast. After a certain period of time, the cells will eventually be inhibited by the pharmaceutical composition until they died, which is directly related to the amount of the pharmaceutical composition,. With respect to fibroblasts that have grown and multiplied before the addition of the pharmaceutical composition, when the pharmaceutical composition is added, it takes time for the pharmaceutical composition to function, and the effect becomes more and more obvious over time.

It is determined in human fibroblasts that the pharmaceutical composition of the present invention can inhibit human fibroblast in vitro, and significantly inhibit human fibroblast growth.

### DESCRIPTION OF THE DRAWINGS

Figure 1: comparison between the pharmaceutical composition well and the control well in the total particle count.
Figure 2: comparison between the pharmaceutical composition well and the control well in the total cell count.
Figure 3: comparison between the pharmaceutical composition well and the control well in the fibroblast count.
Figure 4: comparison between the pharmaceutical composition well and the control well in the viable fibroblast count.
Figure 5: comparison of growth profiles between 3 units of the pharmaceutical composition prepared in Example 1 and 4 units of the pharmaceutical composition prepared in Example 1.
Figure 6: comparison of growth profiles among 3 units of the pharmaceutical composition prepared in Example 1, 4 units of the pharmaceutical composition prepared in Example 1 and the control group.
Figure 7A: in Example 3, there was no fibroblast growth at Day 40 after the culture of the test group.
Figure 7B: in Example 3, there was still a lot of fibroblasts growing at Day 40 after the culture of the control group.
Figure 8A: in the test group of Example 4, there was no fibroblast proliferation in the myocardial cell.
Figure 8B: in the control group of Example 4, myocardial fibrosis was obvious, and fibroblasts proliferated massively.
Figure 9A: in the test group of Example 4, there was no fibroblast proliferation in the marrow.
Figure 9B: in the control group of Example 4, marrow fibrosis was obvious, and fibroblasts proliferated massively.
Figure 10: comparison of growth profiles among 3 units of the pharmaceutical composition prepared in Example 8, 4 units of the pharmaceutical composition prepared in Example 8 and the control group.
Figure 11A to 11C: pictures of gastric mucosa taken by SB capsule endoscopy before the Case 1 was administrated with the pharmaceutical composition (Figure 11A), 9 months after the Case 1 was administrated with the pharmaceutical composition (Figure 11B), and 4 years after the Case 1 was administrated with the pharmaceutical composition (Figure 11C) in Example 9.

### DETAILED DESCRIPTION OF THE INVENTION

The present invention is further illustrated by the following examples in combination with the figures, which should not be construed as a limitation to the present invention. Specific materials and sources thereof used in the embodiments of the present invention are provided below. However, it should be understood that these are merely exemplary and are not intended to limit the present invention. Materials that are the same as or similar to the following reagents and instruments in type, model, quality, properties or function can also be used in the embodiment of the present invention. Unless otherwise specified, the experimental methods used in the following examples are conventional methods, and the materials, reagents and the like used in the following examples are commercially available.

### Example 1: Preparation of the pharmaceutical composition

The pharmaceutical composition was prepared according to the content disclosed in Examples 1 and 2 of Chinese Patent ZL 02105541.6.

Briefly, step 1: the refined sesame oil and *Scutellaria baicalensis* (100 kg: 5kg) were added to a reaction tank and heated. Heating was stopped when the temperature reached 120°C, and the mixture was kept warm for 50 minutes with stirring. The mixture was filtrated to remove the dregs, the obtained extraction was the medicinal oil I.

Step 2: the medicinal oil I was added to another reaction tank and heated. When the temperature reached 85°C, the refined beeswax was added at a ratio of 93 kg of medicinal oil I : 7 kg of beeswax, and stirred well. Heating was stopped when the temperature reached 120°C, kept stirring the warm mixture for 20 minutes to obtain the medicinal oil II.

Step 3: the medicinal oil II was grinded using a colloid mill with a pitch of 0.6 to 0.8 mm and an output speed of 15 Kg/15 min. Alternatively, the medicinal oil II could also be homogenized at 40±2°C for 15 to 20 minutes using a homogenizer with a rotate speed of 6000 to 10000 rpm. The homogenate was stirred at 100 rpm, vacuumized to below 0.09 MP, cooled to 40±2°C, and kept warm for 50 minutes. When the temperature decreased to 20°C and the vacuum degree reached 0.6 to 0.8 MP, the mixture was kept for 20 minutes to obtain the pharmaceutical composition.

According to Example 2 of Chinese Patent ZL 02105541.6, the active ingredients of the pharmaceutical composition prepared by the above method are shown as follows:

| Ingredients | Content per 100 g |
|---|---|
| Natural vitamin E | 15 mg ~ 50 mg |
| Total flavone | 20 mg ~ 60 mg |
| β-sitosterol | 0.20 g - 1.0 g |
| Linoleic acid | 35 g - 55 g |
| Oleic acid | 25 g - 45 g |

### Example 2: Verification of the inhibition effect of the pharmaceutical composition on fibroblast

### 1. Materials and Methods

### 1.1 Instruments, devices, materials and reagents

Quantitative imaging analytical flow cytometry (Amnis^{®}ImageStream*^{x}*MarkII, Merk Millipore, USA, provided by High-throughput Single Cell Analysis Platform of Phoenix Project of Peking University); ultrapure water system (Milli-Q, Millipore, USA); two-stage reverse osmosis purified water system (Beijing Innogreen Technology Co., Ltd.); electronic scale (AB135-S, Mettler-Toledo, Switzerland); electronic scale (ES-1000HA, Changsha Xiangping Technology Development Co., Ltd.); high-speed refrigerated centrifuge (J20-XP, Beckman-Coulter, USA); desktop high speed refrigerated centrifuge (1-15K, Sigma, Germany); biological clean bench (BCN-1360B, Beijing HDL Apparatus Co., Ltd.); CO₂ incubator (Forma3111, Thermo Fisher Scientific, USA); hybridization oven (Maxi14, Thermo Fisher Scientific, USA); particle ice machine (SIM-F124, Sanyo, Japan); electronic constant temperature water bath (CS501-3C type, Chongqing Sida Experimental Instrument Co., Ltd.), drying oven (Chongqing Sida Experimental Instrument Co., Ltd.); inverted microscope *(Nikon* TE2000U, Nikon, Japan); microscopic imaging system *(Nikon* DXM 1200, Nikon, Japan); ordinary optical microscope (BK1201, Chongqing Optical Instrument Factory); micro-sampler (1000 µl, 200 µl, 20 µl, 10 µl, Gilson, France); 6-well culture plate; 15 ml centrifuge tube; 1 ml and 0.5 ml Eppendorf centrifuge tube; dripper; sterilized small surgical instrument; sterilized small beaker; sterilized small flask with lid; sterilized culture dish (ϕ5cm, ϕ6cm); 0.22 µm microporous membrane; needle filter; capped triangular flask; capped small test tube; needle; DMEM medium (GIBCO, Invitrogen Corporation, USA, packaged by Beijing Xinjingke Biotechnology Co., Ltd.); 7-AAD staining solution (provided by High-throughput Single Cell Analysis Platform of Phoenix Project of Peking University); super fetal bovine serum (Hangzhou Sijiqing Biological Engineering Material Co., Ltd.); type I mouse tail collagen (Cat. No. C8062, Solarbio Co.); PBS (self-made, stored at 4°C, and used in an ice bath); Hank's balanced salt solution (self-made, stored at 4°C, and used in an ice bath); 75% ethanol (self-made); 8% Na₂S (self-made); penicillin sodium and streptomycin sulfate for injection; trypsin-EDTA cell digest solution (self-made); portable refrigerator.

### 1.2 Method^{[6-9]}

1) Each well of two 6-well plates was coated with 60 µl of type I mouse tail collagen. The plate was laid flat, sealed with tape, and placed in a stainless steel box. The box was placed in the Maxi14 hybridization oven at 37°C overnight, and stored at 4°C.
2) A Kunming suckling mouse (5 days old, purchased from Laboratory Animal Breeding Base of Academy of Military Medical Sciences) was placed on a foam board, and wiped with a cotton ball (dipped with 8% Na₂S by tweezers) several times to remove hair, especially trunk hair.
3) The mouse was rinsed with deionized water, and killed by breaking its neck.
4) The mouse was placed in 75% ethanol for 2 min, 75% ethanol for 5min, and turned over frequently.
5) The suckling mouse was taken out, and placed in a sterile plate after the ethanol was removed completely.
6) The skin, especially trunk skin, was cut as much as possible. The collected skin was placed in another sterile plate.
7) The sterile skin was spread in the plate with the dermis upward. Tissues such as subcutaneous fat were removed by ophthalmic tweezers.
8) The skin was placed in a sterile flask with lid, and added with 10 ml of cold PBS, 60 µl of penicillin and 60 µl of streptomycin. The skin was turned, stirred and rinsed well.
9) The above step was repeated 4 times with a total of 5 times.
10) The skin was washed with cold Hank's solution + two antibiotics twice.
11) The above suckling mouse skin (washed 7 times) was placed in the sterile flask with lid, added with 10% FBS DMEM medium (cold), and cut into skin grafts later.
12) The 6-well plate (coated with type I mouse tail collagen on the previous day) was washed with cold PBS 3 times. PBS was removed, and each well was added with 1 ml of 10% FBS DMEM medium. The 6-well plate was shaked and placed still.
13) The skin obtained in Step 11 was placed in a sterile plate, and cut into full-thickness skin grafts (about 1 mm²) by sterile ophthalmic curved scissors (note: the skin grafts were obtained, and moisturized by the culture medium). The plate was covered, and should not let it dry.
14) The 10% FBS DMEM medium was removed from the wells of the plate in Step 12 (note: some medium was remained on the bottom of the well as the nutrition for the pre-adherence of graft).
15) The skin grafts obtained in Step 13 were averagely placed into each well (A1, A2, A3, B1, B2, B3) of the two 6-well plates. About 10 pieces of skin grafts were placed into one well by straight tweezers under microscope. The skin graft was spread by straight tweezers under microscope, and pressed to adhere closely. Each skin graft was located in the center of the well, and the area of the spread graft in each well was equal (adjustment was carried out if there was a difference). After checking the adherence of each graft, the plate was covered and sealed with tape except for one side (for the entrance of CO₂).
16) The freshly formulated 10% FBS DMEM medium (stored in a refrigerator) was incubated in a water bath at 37°C for 10 min to avoid the expansion and contract of the skin graft, which would cause the skin graft falling from the bottom of the well.
17) The plate containing the skin grafts was incubated in an incubator at 37°C, 5% CO₂ for 4 h. 4 ml of the above incubated 10% FBS DMEM medium was slowly added dropwise from the rim to each well. De-adherence of the skin graft was not observed.
18) On Day 5 of the experiment, all skin grafts in all wells were well adhered, and recorded by photograph. The test pharmaceutical composition was added to the wells. Specifically, 3 units (about 0.08 g per unit) of pharmaceutical composition, 4 units of pharmaceutical composition or equal amount of medium (as control) was added to the corresponding well, and 3 ml of 10% FBS DMEM medium incubated at 37°C was added to each well at the same time.
19) The medium was changed regularly. Generally, 3 ml of original medium was removed, and an equal amount of 10% FBS DMEM medium incubated at 37°C was added.
20) The growth of fibroblasts was observed and recorded regularly with the *Nikon* TE2000U inverted microscope during the whole culture process. Photos were recorded with the *Nikon* DXM1200 micro-camera and saved on a computer. Pictures at certain time points were selected and analyzed with *Image-Pro Plus* professional image software to draw the growth profile of fibroblast.
21) The experiment was stopped after a certain period of cultivation. Cells were collected from one control well, one well with 3 units of pharmaceutical composition and one well with 4 units of pharmaceutical composition. The cells were detected and analyzed with a flow cytometer. The skin grafts and supernatant were removed from the well. The well was rinsed with cold PBS 3 times. 0.5 ml of trypsin and 0.5 ml of EDTA cell digest solution were added to the well, shaked and reacted at room temperature for a certain time. The reaction was stopped when cell morphology change was observed under the inverted microscope. The well was added with 1 ml of 10% FBS DMEM medium to stop the reaction completely, followed by addition of 3 ml of cold Hank's solution. The bottom of the well was piped repeatedly until the cells were completely separated from the bottom of the well. The obtained cell suspension was added to a 15 ml centrifuge tube, and centrifuged at 4°C, 2000 rpm for 5 min. The supernatant was removed, and the volume of the residues was about 0.5 ml. The cells was suspended, added to an Eppendorf tube, and centrifuged at 4°C, 2000 rpm for 5 min. The supernatant was removed, and the volume of the residue was about 25 µl. It should be noticed that the above steps were carried out in an ice bath, and the obtained cells were labeled accurately. The cells were placed in the portable refrigerator with ice packs, and immediately tested at Peking University. At the flow cytometer room of Peking University, the cells were piped evenly, added with 10 µl of 7-AAD, mixed well and tested immediately. Detection parameters: single laser wavelength of 488 nm, 150mW; SSC: 0.5 mV (785 nm); 40x objective lens.

### 2. Results

### 2.1 Test results of quantitative imaging analytical flow cytometry

### 2.1.1 Total particle count

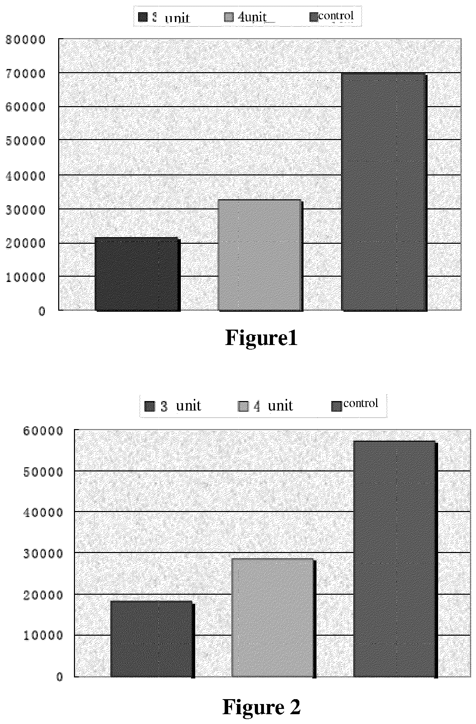

All the particles in the cell suspension to be tested were counted to obtain the absolute number of particles. The maximum number of counted particles was set to 300,000. The counted particles included viable cells, dead cells and non-cells. The shape of each particle could be observed clearly through the image recorded by the instrument. Although the total particle count does not accurately reflect the true number of cells, it is of great reference significance. When analyzing the results, the non-cellular components contained therein should be fully considered. It can be seen from the test results that:
The cells were collected and tested by the flow cytometry to obtain a scatter diagram, which was analyzed by IDEAS software (published by Amnis and matched with the flow cytometer) to obtain the total number of particles. The results are shown in Figure 1. Compared with the control well, the total number of particles varies greatly, and the pharmaceutical composition well has a significantly decreased total number of particles, indicating that the pharmaceutical composition obviously regulated the growth of fibroblast. The effect may vary in a certain extent according to the dosage of the pharmaceutical composition.

### 2.1.2 Fibroblast count

In addition to the total number of particles, the scatter diagram obtained by the flow cytometry was further analyzed by IDEAS software (published by Amnis and matched with the flow cytometer) to obtain the total number of cells, the number of fibroblasts, and the number of viable fibroblasts. In this Example, the parameters were reset, wherein the parameters included two important parameters of aspect ratio and area. The particles that did not meet the cell parameters were excluded to screen the particles that met the cell parameters out of the total particles. The results obtained represented the total number of different types of cells. The results are shown in Figure 2. Compared with the control well, the total number of cells varies greatly, and the pharmaceutical composition well has a significantly decreased total number of cells, indicating that the pharmaceutical composition obviously regulated the growth of fibroblast. The effect may vary in a certain extent according to the dosage of the pharmaceutical composition.

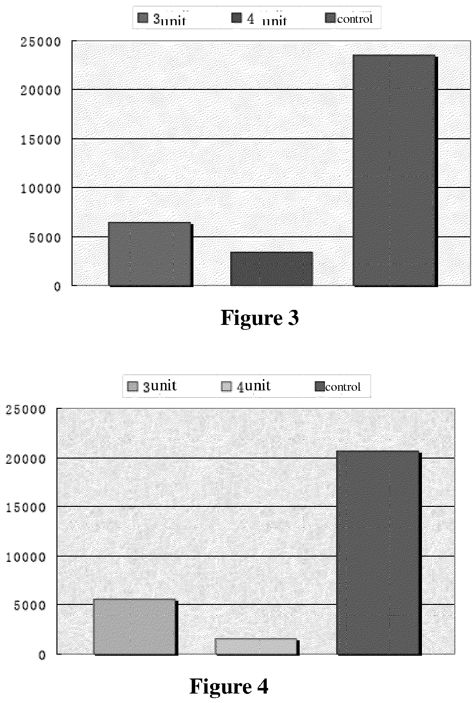

The non-fibroblasts were excluded to screen the cells that met the fibroblast parameters out of the total cells and obtain the total number of fibroblasts. The results are shown in Figure 3. Compared with the control well, the total number of cells varies greatly, and the pharmaceutical composition well has a significantly decreased total number of cells, indicating that the pharmaceutical composition obviously regulated the growth of fibroblast. The effect may vary in a certain extent according to the dosage of the pharmaceutical composition.

The final step of the flow cytometry test was to detect the number of viable cells among the total number of fibroblasts to reflect the effect of different analytes on fibroblast activity and life span. This was achieved by staining the fibroblasts with 7-AAD staining solution. The results are shown in Figure 4. Compared with the control well, the number of viable cells varies greatly, and the pharmaceutical composition well has a significantly decreased total number of cells, indicating that the pharmaceutical composition obviously regulated the growth of fibroblast. The effect may vary in a certain extent according to the dosage of the pharmaceutical composition.

### 2.2 Drawing cell growth profile with the number of fibroblasts at different time points

Three areas with high, medium and low cell density were selected from each well of the plate. Three fields of vision with the same area were randomly selected from each area, with a total of 9 fields of vision with the same area. Cells with typical morphology were counted to obtain the total number of cells. Cells were counted by the professional image processing software *Image-Pro plus* Version6.0. Cell count was carried out by clicking the "Measure" button in the menu, selecting the "Measurements..." item in the drop-down menu, and selecting the "Create point feature" tool in the "Features" toolbar in the dialog box. A total of 12 time points (167 h, 186 h, 197 h, 210 h, 222 h, 234 h, 247 h, 258 h, 269 h, 281 h, 291 h and 300 h after the experiment) were selected in the experiment. Fibroblast count results are shown in Table 1:

**Table 1**

| Additive (right) | 3 units | 4 units | Control |
|---|---|---|---|
| Test time points (below) | | | |
| 1 | 27 | 54 | 378 |
| 2 | 51 | 54 | 432 |
| 3 | 39 | 34 | 432 |
| 4 | 57 | 30 | 420 |
| 5 | 33 | 21 | 369 |
| 6 | 39 | 15 | 447 |
| 7 | 24 | 15 | 477 |
| 8 | 30 | 21 | 396 |
| 9 | 27 | 12 | 408 |
| 10 | 12 | 9 | 447 |
| 11 | 15 | 15 | 459 |
| 12 | 18 | 12 | 450 |

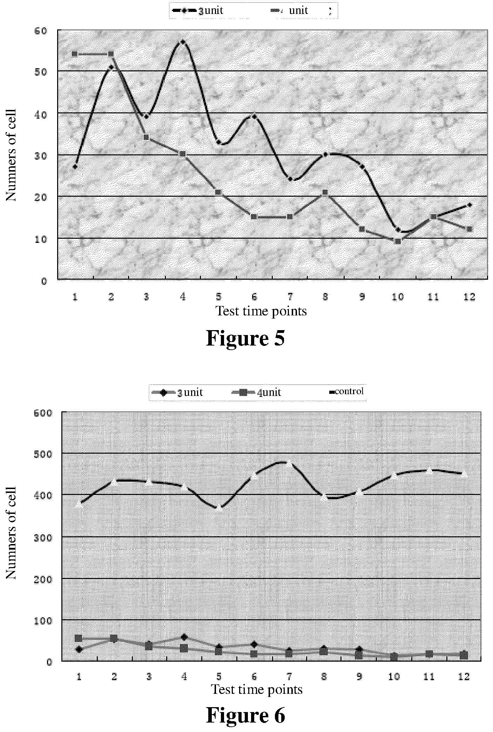

The fibroblast growth profile is drawn based on the above results. For comparison, the growth profile is expressed as different line charts. The results show that there are a large number of cells in the control well, and the overall trend of the growth profile is upward, indicating that the cells increas over time, and the cell growth is not affected. There are a small number of cells in the pharmaceutical composition well, and the overall trend of the growth profile is downward, indicating that the cells decrease over time. In addition to the obviously inhibited cell growth, the viable cells decrease over time as well, indicating that the cell growth is inhibited. Specific results are shown in Figure 5 and Figure 6. It can be seen from the comparison of the growth profiles of 3 units of pharmaceutical composition and 4 units of pharmaceutical composition that the overall trend of the growth profile is obviously downward, and the correlation between the two is good. It can be seen from the comparison of the growth profiles of 3 units of pharmaceutical composition, 4 units of pharmaceutical composition and the control group that the number of cells varies greatly. The overall trend of the first two is downward, while the overall trend of the latter is upward.

### 2.3 Microscopic examination at different time points

A total of 14 detection time points for fibroblast growth (48 h, 66 h, 167 h, 186 h, 197 h, 210 h, 222 h, 234 h, 247 h, 258 h, 269 h, 281 h, 291 h and 300 h after the experiment) were selected to visually show the in vitro growth of fibroblast of suckling mouse skin graft under the effect of different biologically active substances. Similar to the results of the previous experiment, it can also be seen under the microscope that the pharmaceutical composition has a significant inhibition effect on fibroblast growth, while the fibroblasts in the control group without the pharmaceutical composition grow vigorously. The results under the microscope show that the pharmaceutical composition has a very significant inhibition effect on fibroblast. Fibroblasts growing out of the grafts are significantly inhibited by the pharmaceutical composition, and changed in morphology. The cells turn black and shrink until they die and dissolve. The effect of the pharmaceutical composition on fibroblasts that just grow out of the skin graft can also be observed under the microscope. In fact, the cells are inhibited at the early stage of growth without a chance of diffusion and growth. With respect to a large amount of fibroblasts growing in a short period of time, the cells will not be immediately inhibited or die due to the temporary balance of the pharmaceutical composition and fibroblast. After a certain period of time, the cells will eventually be inhibited by the pharmaceutical composition until they die, which is directly related to the amount of the pharmaceutical composition. With respect to fibroblasts that have grown and multiplied before the addition of the pharmaceutical composition, when the pharmaceutical composition is added, it takes time for the pharmaceutical composition to function, and the effect becomes more and more obvious over time. Specific results are shown in Table 2 below:

**Table 2**

| | 3 units | 4 units | Control |
|---|---|---|---|
| Time points | | | |
| 1 | Fibroblast growth is not found at the edge of the graft | Fibroblast growth is not found at the edge of the graft | Fibroblast growth is not found at the edge of the graft |
| 2 | Fibroblast growth is found at the edge of the graft | Fibroblast growth is not found at the edge of the graft | Fibroblast growth is found at the edge of the graft |
| 3 | There are a few fibroblasts growing at the edge of the graft | There are very few fibroblasts at the edge of the graft | There are a large number of fibroblasts with obvious growth |
| 4 | There are a few fibroblasts at the edge of the graft | There are very few fibroblasts | Fibroblasts grow vigorously away from the graft, and densely arrange with a typical morphology |
| 5 | There are fibroblasts growing around the edge of the graft | There are very few fibroblasts | A large number of fibroblasts gow away from the graft, and densely arrange with a typical morphology |
| 6 | There are few fibroblasts, which are located around the graft with a typical morphology | There are very few fibroblasts | Fibroblasts grow away from the graft, and densely arrange with a typical morphology |
| 7 | There are very few fibroblasts with a fairly good growth condition, which is close to the periphery of the graft and failed to expand further | There are very few fibroblasts, which fail to grow further into the distance | Fibroblasts very densely arrange with a typical morphology, and grow away from the graft, contact inhibition is formed |
| 8 | There are very few fibroblasts, which are located around the graft and failed to expand further | There are very few viable fibroblasts, which are located around the graft | Fibroblasts very densely arrange with a typical morphology |
| 9 | There are a few viable fibroblasts in an aging state with a changed morphology, which are located around the graft | There are a few viable fibroblasts in an aging state with a nontypical morphology, which are located around the graft | Fibroblasts densely arrange with a typical morphology, and grow away from the graft |
| 10 | There are very few viable fibroblasts, which are located around the graft | There are very few fibroblasts, which are merely located around the graft and fail to expand to the distance | Fibroblasts densely arrange with a typical morphology, and grow away from the graft |
| 11 | There are a few fibroblasts, which are located around the graft and fail to expand to the distance, the growth is significantly inhibited | There are a few degenerated and dead fibroblasts, which are located around the graft and fail to expand, the growth is significantly inhibited | Fibroblasts very densely arrange with a typical morphology, and grow away from the graft, the growth is at its best |
| 12 | There are a few fibroblasts with a changed morphology, which are located around the graft, the growth is significantly inhibited | There are very few fibroblasts with a nontypical morphology, which are located around the graft, the growth is significantly inhibited | Fibroblasts densely arrange with a typical morphology, and grow away from the graft |
| 13 | There are a few fibroblasts, which are located around the graft, the growth is significantly inhibited | There are very few fibroblasts, which are located around the graft, the growth is significantly inhibited | Fibroblasts densely arrange with a typical morphology, and grow vigorously away from the graft |
| 14 | Fibroblasts are located around the graft, the morphology is changed, the fibroblasts are dying, indicating that the cell growth is significantly inhibited | There are very few fibroblasts, which have already dead, the growth is significantly inhibited | Fibroblasts densely arrange with a typical morphology, and grow vigorously away from the graft |

### Example 3: Inhibition effect of the pharmaceutical composition on human fibroblast

### 1. Materials and Methods

### 1.1 Instruments, devices, materials and reagents

Ultrapure water system (Milli-Q, Millipore, USA); two-stage reverse osmosis purified water system (Beijing Innogreen Technology Co., Ltd.); electronic scale (AB135-S, Mettler-Toledo, Switzerland); electronic scale (ES-1000HA, Changsha Xiangping Technology Development Co., Ltd.); high-speed refrigerated centrifuge (J20-XP, Beckman-Coulter, USA); desktop high speed refrigerated centrifuge (1-14K, Sigma, Germany); biological clean bench (BCN-1360B, Beijing HDL Apparatus Co., Ltd.); CO₂ incubator (Forma3111, Thermo Fisher Scientific, USA); particle ice machine (SIM-F124, Sanyo, Japan); electronic constant temperature water bath (CS501-3C type, Chongqing Sida Experimental Instrument Co., Ltd.), drying oven (Chongqing Sida Experimental Instrument Co., Ltd.); inverted microscope *(Nikon* TE2000U, Nikon, Japan); microscopic imaging system *(Nikon* DXM 1200, Nikon, Japan); ordinary optical microscope (BK1201, Chongqing Optical Instrument Factory); micro-sampler (1000 µl, 200 µl, 20 µl, 10 µl, Gilson, France); 12-well culture plate; 15 ml centrifuge tube; 1 ml and 0.5 ml Eppendorf centrifuge tube; dripper; sterilized small surgical instrument; sterilized small beaker; sterilized small flask with lid; sterilized culture dish (ϕ5cm, ϕ6cm); 0.22 µm microporous membrane; needle filter; capped triangular flask; capped small test tube; needle; DMEM medium and 1640 medium (GIBCO, Invitrogen Corporation, USA, packaged by Beijing Xinjingke Biotechnology Co., Ltd.); fetal bovine serum (FBS, Hangzhou Sijiqing Biological Engineering Material Co., Ltd.); PBS (self-made, stored at 4°C); 75% ethanol (self-made); trypsin-EDTA cell digest solution (self-made); portable refrigerator; vortex shaker.

### 1.2 Methods and results

### 1.2.1 Early human embryonic tissue (disclosed for illustration purposes and does not form part of the claimed invention)

Source and transportation of the tissue: with the informed consent of the patient, early human embryonic tissue was obtained from the hospital through artificial abortion. The human embryonic tissue was placed in 10% FBS DMEM complete medium in an ice bath, and immediately processed in the laboratory.

Graft culture: A small amount of tissue was cut into small pieces. The tissue was washed with PBS once, then washed with a high concentration of double antibiotics-PBS five times. The tissue was rinsed with 15% FBS 1640 medium once, and cut into grafts (1 mm³). The grafts were cultured in a 12-well plate. Each well comprised several pieces of grafts, and was added with a small amount of 15% FBS1640 medium around. The plate was left to stand in the incubator at 37°C, 5% CO₂ for 2.5 h. Each well was added with 2 ml of 15% FBS1640 medium. The grafts were divided into two groups: test group and control group. The test group was added with two units (0.1 g per unit) of the pharmaceutical composition prepared in Example 1, and the control group was added with the same amount of medium. The plate was incubated in the incubator at 37°C, 5% CO₂. The cell growth in the test group and control group was observed after a certain period of time.

Results: On Day 70 of the cultivation, no cell growth was observed for the test group, while there was a large number of fibroblasts growth for the control group. Conclusion: The pharmaceutical composition inhibits human fibroblast.

Single cell culture: A small amount of tissue was cut into small pieces. The tissue was washed with PBS once, then washed with a high concentration of double antibiotics-PBS five times. The tissue was rinsed with 15% FBS 1640 medium once, and cut into grafts (1 mm³). The grafts were placed in a 50 ml centrifuge tube, and added with a mixed solution of 2 ml of 0.25% trypsin and 2 ml of 0.02% EDTA. The tube was shaked in a constant temperature shaker at 37°C for 35 min, and vortexed for an appropriate time. The solution was filtrated through a stainless steel filter (80 mesh). The filtrate was added with an appropriate amount of PBS, and centrifuged at 1500 rpm for 7 min. The supernate was removed, and the residues were added with PBS, and centrifuged at 1700 rpm for 5 min. The supernate was removed, and the residues were added with 8 ml of 15% FBS1640 medium. The cells were counted, and cultured in a 12-well plate (2 ml of cell suspension per well). The cells were divided into two groups: test group and control group. The test group was added with two units (0.1 g per unit) of the pharmaceutical composition prepared in Example 1, and the control group was added with the same amount of medium. The plate was incubated in the incubator at 37°C, 5% CO₂. The cell growth in the test group and control group was observed after a certain period of time.

Results: On Day 70 of the cultivation, no cell growth was observed for the test group, while there was a large number of fibroblasts growth for the control group. Conclusion: The pharmaceutical composition inhibits human fibroblast growth.

### 1.2.2 Gastric fundus cancer tissue

Source and transportation of the tissue: with the informed consent of the patient, gastric fundus cancer tissue was obtained from the hospital through surgical excision. The gastric fundus cancer tissue was placed in 10% FBS DMEM complete medium in an ice bath, and immediately processed in the laboratory.

Gastric fundus cancer tissue graft culture: A small amount of cancer core tissue was cut into small pieces. The tissue was washed with PBS once, then washed with a high concentration of double antibiotics-PBS five times. The tissue was rinsed with 15% FBS 1640 medium once, and cut into grafts (1 mm³). The grafts were cultured in a 12-well plate. Each well comprised several pieces of grafts, and was added with a small amount of 15% FBS1640 medium around. The plate was left to stand in the incubator at 37°C, 5% CO₂ for 2.5 h. Each well was added with 2 ml of 15% FBS1640 medium. The grafts were divided into two groups: test group and control group. The test group was added with two units (0.1 g per unit) of the pharmaceutical composition prepared in Example 1, and the control group was added with the same amount of medium. The plate was incubated in the incubator at 37°C, 5% CO₂. The cell growth in the test group and control group was observed after a certain period of time.

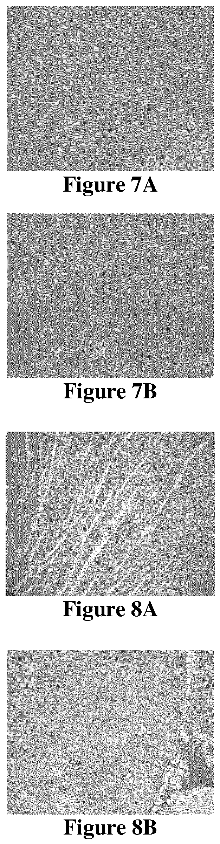

Results: No fibroblast growth was observed for the test group (Figure 7A), while there was a large number of fibroblasts growth in the control group (Figure 7B). Conclusion: The pharmaceutical composition inhibits human fibroblast growth.

### Example 4: Inhibition effect of the pharmaceutical composition on organ fibrosis

### 1. Materials and Methods

### 1.1 Instruments, devices, materials and reagents

The laboratory animals were 10-month-old Wistar male rats purchased from Institute of Laboratory Animal Sciences, CAMS. The rats were divided into two groups (59 rats for the test group, and 27 rats for the control group). After adapting the feeding environment for 3 days, the rats were subjected to differentiated feeding. The control group was continuely fed with the general nutrient feed produced by Institute of Laboratory Animal Sciences, CAMS, and the test group was fed with the nutrient feed added with the pharmaceutical composition prepared in Example 1. The first batch of rats (5 rats for the test group, and 5 rats for the control group) which were fed with the nutrient feed comprising the pharmaceutical composition and the normal nutrient feed were euthanized after 526 days of feeding. The second batch of rats (18 rats for the test group, and 10 rats for the control group) which were fed with the nutrient feed comprising the pharmaceutical composition or the normal nutrient feed were euthanized after 623 days of feeding. Samples of various tissues and organs (heart, liver, lungs, kidneys, and bone marrow) were sliced and stained (hematoxylin-eosin staining, HE staining).

Preparation method of the nutrient feed comprising the pharmaceutical composition: normal nutrient feed: pharmaceutical composition=9:1 (mass ratio). 9 parts of normal nutrient feed were added to a mixer. 1 part of the pharmaceutical composition was properly heated, stirred well, and poured into the mixer. The mixer was covered with a cap and started to run. The normal nutrient feed and the pharmaceutical composition were mixed evenly, and then screened through a fine sieve. The sieve was shaken vigorously to further mix the feed evenly. Finally, the mixture was processed into a biscuit feed with a feed processing machine. The feed was spreaded on a stainless steel plate, and dried in a steam drying room at 80°C for 4 hours. The feed was cooled naturally after the steam stopped.

### 2. Results

With regard to myocardial fibrosis of aging rat, there are significant differences between the test group and the control group. Among the 17 samples of the test group, 7 samples have myocardial fibrosis, with an abnormal rate of 41%. Among the 16 samples of the control group, 13 samples have myocardial fibrosis, with an abnormal rate of 81%. The test group is superior to the control group, indicating that the pharmaceutical composition inhibits myocardial fibrosis (see Figure 8A and Figure 8B for exemplary tissue pictures).

With regard to liver fibrosis of aging rat, there are significant differences between the test group and the control group. Among the 18 samples of the test group, 3 samples have fibrosis, with an abnormal rate of 17%. Among the 17 samples of the control group, 10 samples have fibrosis, with an abnormal rate of 59%. The test group is significantly superior to the control group. Bile duct fibrosis is a typical indicator of liver aging. The results indicate that the pharmaceutical composition has a significant effect of inhibiting liver fibrosis and promoting liver regeneration and recovery.

With regard to lung tissue fibrosis of aging rat, it is determined that the pharmaceutical composition significantly inhibits the lung tissue fibrosis of aging rat. The lung tissue fibrosis rate in the test group is 5/20=25%, while the lung tissue fibrosis rate in the control group is 10/14=71%.

With regard to renal parenchymal fibrosis of aging rat, there are significant differences between the test group and the control group. Among the 19 samples of the test group, 5 samples have fibrosis, with an abnormal rate of 26%. Among the 16 samples of the control group, 11 samples have fibrosis, with an abnormal rate of 69%.

The test group is significantly superior to the control group.

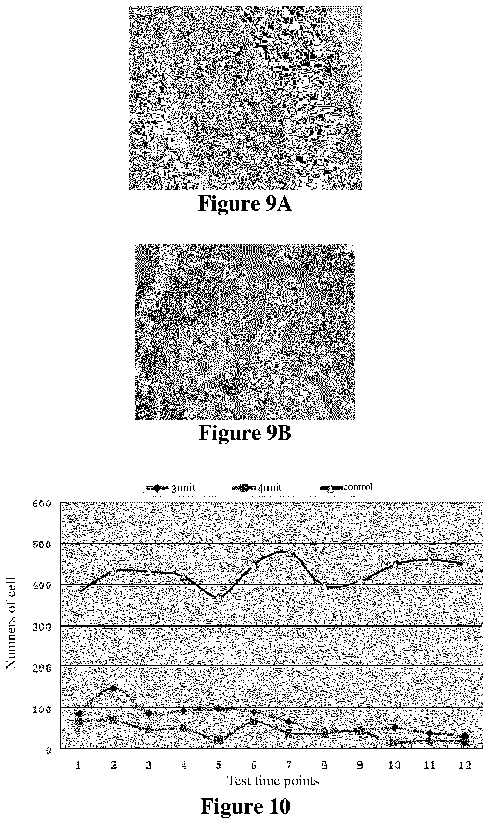

The pharmaceutical composition inhibits the bone marrow fibrosis of aging rat. None of the 6 samples of the test group have bone marrow fibrosis, while all 6 samples of the control group have fibrosis. In the test group, myeloid cells are densely arranged, and erythroid precursor cells, myeloid (granulocyte) precursor cells and megakaryocytes can be easily found (see Figure 9A). In the control group, fibroblasts significantly proliferate, and fibrous tissues are generated (see Figure 9B). The specific results are shown in Table 3 below:

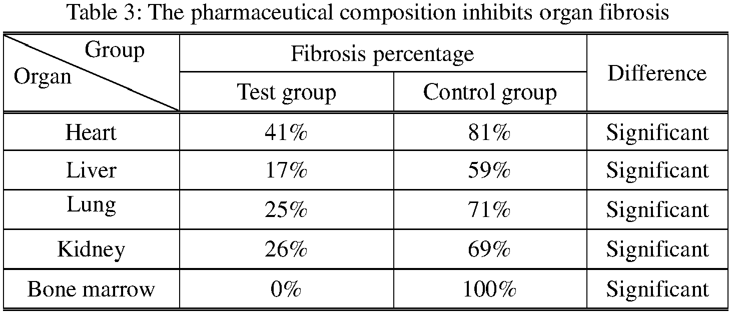

### Example 5: Preparation of the pharmaceutical composition comprising Scutellaria baicalensis and Cortex Phellodendri as well as the effect thereof on regulating fibroblast growth

The pharmaceutical composition was prepared according to the method of Example 1, wherein the refined sesame oil, *Scutellaria baicalensis* and *Cortex Phellodendri* (100 kg: 5 kg: 4 kg) were added to a reaction tank in step 1, and other steps were the same as in Example 1.

The pharmaceutical composition obtained in this example was subjected to the test methods of Example 2, 3 and 4. The results are similar to those of the pharmaceutical composition obtained in Example 1, indicating that this pharmaceutical composition can significantly regulate fibroblast growth, and inhibit human fibroblast and organ fibrosis of aging rat.

### Example 6: Preparation of the pharmaceutical composition comprising Scutellaria baicalensis and Coptis chinensis as well as the effect thereof on regulating fibroblast growth

The pharmaceutical composition was prepared according to the method of Example 1, wherein the refined sesame oil, *Scutellaria baicalensis* and *Coptis chinensis* (100 kg: 5 kg: 4 kg) were added to a reaction tank in step 1, and other steps were the same as in Example 1.

The pharmaceutical composition obtained in this example was subjected to the test methods of Example 2, 3 and 4. The results are similar to those of the pharmaceutical composition obtained in Example 1, indicating that this pharmaceutical composition can significantly regulate fibroblast growth, and inhibit human fibroblast and organ fibrosis of aging rat.

### Example 7: Preparation of the pharmaceutical composition comprising Scutellaria baicalensis, Cortex Phellodendri and Coptis chinensis as well as the effect thereof on regulating fibroblast growth

The pharmaceutical composition was prepared according to the method of Example 1, wherein the refined sesame oil, *Scutellaria baicalensis* and *Coptis chinensis* (100 kg: 5 kg: 5 kg: 5 kg) were added to a reaction tank in step 1, and other steps were the same as in Example 1.

The pharmaceutical composition obtained in this example was subjected to the test methods of Example 2, 3 and 4. The results are similar to those of the pharmaceutical composition obtained in Example 1, indicating that this pharmaceutical composition can significantly regulate fibroblast growth, and inhibit human fibroblast and organ fibrosis of aging rat.

### Example 8: Preparation of the pharmaceutical composition comprising Scutellaria baicalensis, Coptis chinensis, Cortex Phellodendri, Pericarpium Papaveris and earthworm as well as the effect thereof on regulating fibroblast growth

The pharmaceutical composition was prepared according to the method of Example 1, wherein the refined sesame oil, *Scutellaria baicalensis, Coptis chinensis, Cortex Phellodendri, Pericarpium Papaveris* and earthworm (100 kg: 5 kg: 4 kg: 4 kg: 5 kg: 5 kg) were added to a reaction tank in step 1, and other steps were the same as in Example 1.

The pharmaceutical composition obtained in this example was subjected to the test method of Example 2, and the following results are obtained:
1) Drawing cell growth profile with the number of fibroblasts at different time points

There are a large number of cells in the control well, and the overall trend of the growth profile is upward, indicating that the cells increase over time, and the cell growth is not affected. There are a small number of cells in the M well, and the overall trend of the growth profile is downward, indicating that the cells decrease over time. In addition to the obviously inhibited cell growth, the viable cells decrease over time as well, indicating that the cell growth is inhibited (see Figure 10). Specific results of fibroblast growth at different time points are shown in Table 4 below:

**Table 4**

| | 3 units | 4 units | Control |
|---|---|---|---|
| Time point | | | |
| 1 | Fibroblast growth is not found at the edge of the graft | Fibroblast growth is not found at the edge of the graft | Fibroblast growth is not found at the edge of the graft |
| 2 | Fibroblast growth is found at the edge of the graft | Fibroblast growth is not found at the edge of the graft | Fibroblast growth is found at the edge of the graft |
| 3 | There are a few fibroblasts growing at the edge of the graft | There are a few fibroblasts growing significantly at the edge of the graft | There are a large number of fibroblasts with obvious growth |
| 4 | There are a few scattered fibroblasts at the edge of the graft | There are very few fibroblasts | Fibroblasts grow vigorously away from the graft, and densely arrange with a typical morphology |
| 5 | There are a few fibroblasts growing | There are a few fibroblasts with a nontypical morphology, indicating poor cell growth | A large number of fibroblasts grow away from the graft, and densely arrange with a typical morphology |
| 6 | There are a few fibroblasts, which are located around the graft with a typical morphology, and fail to expand further | There are a few fibroblasts, which are located around the graft with a poor cell growth, and fail to expand further | Fibroblasts grow away from the graft, and densely arrange with a typical morphology |
| 7 | There are very few fibroblasts with a fairly good growth condition, which are close to the periphery of the graft and fail to expand further into the distance | There are very few viable fibroblasts, and most of the grown fibroblasts have dead | Fibroblasts very densely arrange with a typical morphology, and grow away from the graft, contact inhibition is formed |
| 8 | There are very few fibroblasts with a normal morphology and fairly good growth condition, which fail to expand further into the distance | There are a few viable fibroblasts, which are located around the graft | Fibroblasts very densely arrange with a typical morphology |
| 9 | There are a few viable fibroblasts in an aging state with a nontypical morphology, which are located around the graft | There are a few viable fibroblasts, which are located around the graft and fail to expand further into the distance | Fibroblasts densely arrange with a typical morphology, and grow away from the graft |
| 10 | The morphology of fibroblast is changed, the fibroblasts stop to grow and fail to expand further into the distance | The morphology of most fibroblasts around the graft is changed, the fibroblast growth is significantly inhibited, and the fibroblast fail to expand further into the distance | Fibroblasts densely arrange with a typical morphology, and grow away from the graft |
| 11 | There are a few fibroblasts, which are located around the graft and fail to expand to the distance, the growth is significantly inhibited | There are degenerated and dead fibroblasts and a few viable fibroblasts, and the growth is significantly inhibited | Fibroblasts very densely arrange with a typical morphology, and grow away from the graft, the growth is at its best |
| 12 | There are a few fibroblasts, which are located around the graft, and the growth is significantly inhibited | There are a few fibroblasts, which are located around the graft, and the growth is significantly inhibited | Fibroblasts densely arrange with a typical morphology, and grow away from the graft |
| 13 | The morphology of fibroblast is changed obviously, and the growth is significantly inhibited | There are a few fibroblasts with a changed morphology, which are located around the graft, and the growth is significantly inhibited | Fibroblasts densely arrange with a typical morphology, and grow vigorously away from the graft |
| 14 | There are very few fibroblasts, which are located around the graft and fail to expand, and the growth is significantly inhibited | There are a few fibroblasts, which fail to expand, and the growth is significantly inhibited | Fibroblasts densely arrange with a typical morphology, and grow vigorously away from the graft |

The pharmaceutical composition obtained in this example was subjected to the test methods of Example 3 and 4. It is found that the pharmaceutical composition prepared in this example can inhibit human fibroblast and organ fibrosis of aging rat.

### Example 9: Clinical cases of regulating fibroblast growth of the pharmaceutical composition

### Case 1: Regeneration and recovery of gastric scar

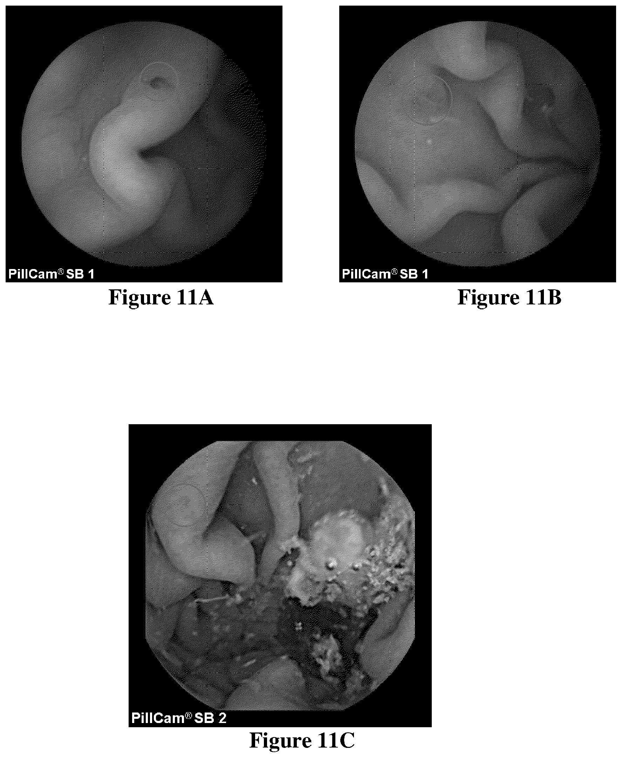

Case 1 (male, born on 25 Feb 1953) has a history of gastric ulcer and chronic erosive gastritis. The patient was administrated with five units each time (0.1 g of the pharmaceutical composition per unit) of the pharmaceutical composition obtained in Example 1 three times a day. Before the administration, SB capsule endoscopy showed obvious scars on the gastric mucosa. After 9 months of administration, it was found that the mucosal scars were significantly reduced. After 4 years of administration, it was found that the mucosal scars were recovered (see Figure 11A to 11C).

### Case 2: Regeneration and recovery of liver fibrosis

Case 2 (male, born on 6 Jul 1950) has a history of chronic hepatitis and liver fibrosis for 30 years, with a spleen 7.4 cm below the ribs. The patient was administrated with five units each time of the pharmaceutical composition obtained in Example 1 three times a day. The patient was recovered to normal after three years. The corresponding biochemical function indexes are normal, which can be seen in Table 5 below:

**Table 5**

| Liver fiberography | | | |
|---|---|---|---|
| | Before the administration | After 3 years of administration | Reference value |
| Hyaluronic acid | 44.2 | 36.0 | Less than 40 ng/mL |
| Type III procollagen | 8.0 | 8.3 | Less than 8.6 ng/mL |
| Type IV collagen | 24.9 | 24.1 | Less than 23.8 ng/mL |
| Laminin | 125 | 107 | Less than 113 µg/L |

### Case 3: Regeneration and recovery of arteriosclerosis (not part of the present invention)

Case 3 (female, born on 12 Feb 1957) has a history of hypertension for 18 years and a history of diabetes for 17 years. The patient was administrated with five units each time of the pharmaceutical composition obtained in Example 1 three times a day. The coronary heart disease and arterial plaque are recovered at present.

Before the administration, the color B-ultrasound of double carotid arteries showed plaque on the left artery (soft plaque). After one year of administration, the ultrasound showed no obvious abnormalities in bilateral carotid arteries.

Before the administration, the coronary spiral CT showed coronary artery calcification and stenosis in the proximal segment of the left anterior descending branch (30%). After one year of administration, there were no abnormalities in coronary arteries diagnosed by imaging.

### References

[1] Cai LI, Current Research of Organ Fibrosis, Basic and Clinic of Organ Fibrosis [M], Editor-in-Chief: Cai LI, Beijing: People's Medical Publishing House, 2003: 1-15.
[2] Tianshui YU, Dawei GUAN, Yong MA, Research Progress of Myofibroblast in Fibrotic Disease [J], Chinese Journal of Histochemistry and Cytochemistry, 2013, 22(2): 167-171.
[3] Mitchell RN, Cotran RS, Tissue repair: cellular growth, fibrosis, and wound healing, In: Cotran RS, Kumar V, Collins T(eds.), Robbin's Pathologic Basis of Disease, W.B. Saunders Company, 1999(6th ed); 89-93.
[4] Wei HE, Dermal Fibroblast Biology, Basics of Modern Dermatology, Editor-in-Chief: Xuejun ZHANG, Weida LIU, Chundi HE, Beijing: People's Medical Publishing House. 2001; 137-152.
[5] Jingqu ZHOU, Qinglian PENG, Inherent Connective Tissue, Histology, Editor-in-Chief: Lingzhong CHENG, Beijing: People's Medical Publishing House, 1981; 218-264.
[6] Translator: Hong ZHOU, Fibroblast Isolation and Culture, Cell Experiment Guide (Volume 1) [M], Translator: Peitang HUANG et al. Beijing: Science Press, 2001: 27-31.
[7] Zheng E, Culture of Individual Tissue and Cell, Tissue Culture and Molecular Cytology Techniques [M], Editor-in-Chief: Zheng E, Beijing: Beijing Publishing House, 1995: 120-138.
[8] Xiuqin WANG, Min WU, Culture of Human Skin Fibroblast, Practical Methods and Techniques of Cell Biology [M], Editor-in-Chief: Jingbo ZHANG, Beijing: United Press of Beijing Medical University and China Union Medical University, 1995: 1-2.
[9] Yuping XIAO, In Vitro Culture of Connective Tissue, Principles and Techniques of In Vitro Culture [M], Editor-in-Chief: Qingshan XUE, Beijing: Science Press, 2001: 442-452.

## Claims

1. Pharmaceutical composition suitable for oral administration for use in the therapeutic treatment or prevention of organ fibrosis, scar formation and/or tissue aging, comprising a homogenous mixture of edible oil, beeswax,β-sitosterol, *Scutellaria baicalensis* or the extract of *Scutellaria baicalensis* containing 0.1 to 0.5% of baicalin, *Cortex Phellodendri* or the extract of *Cortex Phellodendri* containing 0.1 to 1% of obaculactone, *Coptis chinensis* or the extract of *Coptis chinensis* containing 0.1 to 1% of berberine, *Pericarpium Papaveris* or the extract of *Pericarpium Papaveris* containing 0.1 to 1% of narcotoline, and earthworm or earthworm extract containing amino acid; wherein the beeswax in the composition forms microcrystals, the content of the beeswax is 0.5 to 50% and the content of the β-sitosterol is 0.1% to 20% by weight based on the total weight of the composition, the pharmaceutical composition comprises 2 to 5% of *Scutellaria baicalensis* or the extract of *Scutellaria baicalensis* containing 0.1 to 0.5% of baicalin, 2 to 5% of *Cortex Phellodendri* or the extract of *Cortex Phellodendri* containing 0.1 to 1% of obaculactone, 2 to 5% of *Coptis chinensis* or the extract of *Coptis chinensis* containing 0.1 to 1% of berberine, 2 to 10% of *Pericarpium Papaveris* or the extract of *Pericarpium Papaveris* containing 0.1 to 1% of narcotoline, and 2 to 10% of earthworm or earthworm extract containing amino acid, by weight based on the total weight of the composition, preferably said organ comprises heart, liver, lungs, kidneys and bone marrow and is from mammal, preferably a human.

2. Pharmaceutical composition for use according to claim 1, **characterized in that** the content of the β-sitosterol in the pharmaceutical composition is 0.5 to 20% by weight, preferably is 1 to 10% by weight.

3. Pharmaceutical composition for use according to claim 1, **characterized in that** the content of the beeswax in the pharmaceutical composition is 3 to 30% by weight, preferably the content of the beeswax is 5 to 20% by weight, more preferably the content of the beeswax is 6 to 10% by weight.

4. Pharmaceutical composition for use according to any one of claims 1 to 3, **characterized in that** the edible oil in the pharmaceutical composition is corn oil, wheat germ oil, soybean oil, rice bran oil, rapeseed oil, sesame oil or fish oil.

5. Pharmaceutical composition for use according to any of the previous claims, **characterized in that** the pharmaceutical composition further comprises propolis, and the content thereof is 0.1 to 30% by weight.

6. Pharmaceutical composition for use according to any of the previous claims, **characterized in that** the pharmaceutical composition comprises water, and the content thereof is less than or equal to 1% by weight.

7. Pharmaceutical composition for use according to any of the previous claims, **characterized in that** the dosage form of the oral pharmaceutical composition is selected from the group consisting of a tablet, pill, capsule, emulsion, gel, syrup and suspension.

8. Pharmaceutical composition for use according to any of the previous claims, **characterized in that** the *Scutellaria baicalensis* is one or more *Labiatae* plants selected from the group consisting of *Scutellaria viscidula bunge, Scutellaria amoena, Scutellaria rehderiana Diels, Scutellaria ikonnikovii Juz, Scutellaria likiangensis* and *Scutellaria hypericifolia.*

9. Pharmaceutical composition for use according to any of the previous claims, **characterized in that** the the *Cortex Phellodendri* is selected from the group consisting of *Phellodendron chinense Schneid, Phellodendron amurense, Phellodendron chinense Schneid var. omeiense, Phellodendron Schneid var. yunnanense* and *Phellodendron chinense Schneid var. falcutum.*

10. Pharmaceutical composition for use according to any of the previous claims, **characterized in that** the *Scutellaria baicalensis* extract is a *Scutellaria baicalensis* extract obtained in sesame oil, the *Cortex Phellodendri* extract is a *Cortex Phellodendri* extract obtained in sesame oil, and the *Coptis chinensis* extract is a *Coptis chinensis* extract obtained in sesame oil.

11. Pharmaceutical composition for use according to any of the previous claims, **characterized in that** the pharmaceutical composition comprises 7% of beeswax, 1% of sterol, 0.5% of obaculactone, 0.3% of baicalin and 0.5% of berberine by weight based on the total weight of the composition.

12. Pharmaceutical composition for use according to any of the previous claims, **characterized in that** the beeswax has microcrystals with a length of 0.1 to 100 microns, preferably at least two microcrystals of the beeswax in the pharmaceutical composition are polymerized into a microcrystal complex, more preferably said microcrystals of beeswax are sufficiently uniformly dispersed in the edible oil.

## Patentansprüche

1. Pharmazeutische Zusammensetzung, geeignet zur oralen Verabreichung zur Verwendung bei der therapeutischen Behandlung von oder Vorbeugung vor Organfibrose, Narbenbildung und/oder Gewebealterung, umfassend ein homogenes Gemisch aus genießbarem Öl, Bienenwachs, β-Sitosterol, *Scutellaria baicalensis* oder dem Extrakt von *Scutellaria baicalensis,* der 0,1 bis 0,5 % Baicalin enthält, *Cortex Phellodendri* oder dem Extrakt von *Cortex Phellodendri,* der 0,1 bis 1 % Obaculacton enthält, *Coptis chinensis* oder dem Extrakt von *Coptis chinensis,* der 0,1 bis 1 % Berberin enthält, *Pericarpium Papaveris* oder dem Extrakt von *Pericarpium Papaveris,* der 0,1 bis 1 % Narcotolin enthält, und Regenwurm oder Regenwurm-Extrakt, der Aminosäure enthält; wobei das Bienenwachs in der Zusammensetzung Mikrokristalle bildet, der Gehalt des Bienenwachses 0,5 bis 50 % beträgt und der Gehalt des β-Sitosterols 0,1 % bis 20 % nach Gewicht auf der Grundlage des Gesamtgewichts der Zusammensetzung beträgt, die pharmazeutische Zusammensetzung umfassend 2 bis 5 % von *Scutellaria baicalensis* oder dem Extrakt von *Scutellaria baicalensis,* der 0,1 bis 0,5 % Baicalin enthält, 2 bis 5 % von *Cortex Phellodendri* oder dem Extrakt von *Cortex Phellodendri,* der 0,1 bis 1 % Obaculacton enthält, 2 bis 5 % von *Coptis chinensis* oder dem Extrakt von *Coptis chinensis,* der 0,1 bis 1 % Berberin enthält, 2 bis 10% von *Pericarpium Papaveris* oder dem Extrakt von *Pericarpium Papaveris,* der 0,1 bis 1 % Narcotolin enthält, und 2 bis 10 % von Regenwurm oder Regenwurm-Extrakt, der Aminosäure enthält, nach Gewicht auf der Grundlage des Gesamtgewichts der Zusammensetzung, wobei das Organ vorzugsweise Herz, Leber, Lungen, Nieren und Knochenmark umfasst und von einem Säugetier stammt, vorzugsweise einem Menschen.

2. Pharmazeutische Zusammensetzung zur Verwendung nach Anspruch 1, **dadurch gekennzeichnet, dass** der Gehalt des β-Sitosterols in der pharmazeutischen Zusammensetzung 0,5 bis 20 Gewichts-% beträgt, vorzugsweise 1 bis 10 Gewichts-% beträgt.

3. Pharmazeutische Zusammensetzung zur Verwendung nach Anspruch 1, **dadurch gekennzeichnet, dass** der Gehalt des Bienenwachses in der pharmazeutischen Zusammensetzung 3 bis 30 Gewichts-% beträgt, wobei der Gehalt des Bienenwachses vorzugsweise 5 bis 20 Gewichts-% beträgt, wobei der Gehalt des Bienenwachses bevorzugter 6 bis 10 Gewichts-% beträgt.

4. Pharmazeutische Zusammensetzung zur Verwendung nach einem der Ansprüche 1 bis 3, **dadurch gekennzeichnet, dass** es sich bei dem genießbaren Öl in der pharmazeutischen Zusammensetzung um Maisöl, Weizenkeimöl, Sojabohnenöl, Reiskleieöl, Rapsöl, Sesamöl oder Fischöl handelt.

5. Pharmazeutische Zusammensetzung zur Verwendung nach einem der vorstehenden Ansprüche, **dadurch gekennzeichnet, dass** die pharmazeutische Zusammensetzung ferner Propolis umfasst und der Gehalt davon 0,1 bis 30 Gewichts-% beträgt.

6. Pharmazeutische Zusammensetzung zur Verwendung nach einem der vorstehenden Ansprüche, **dadurch gekennzeichnet, dass** die pharmazeutische Zusammensetzung Wasser umfasst und der Gehalt davon weniger als oder gleich 1 Gewichts-% beträgt.

7. Pharmazeutische Zusammensetzung zur Verwendung nach einem der vorstehenden Ansprüche, **dadurch gekennzeichnet, dass** die Dosierungsform der oralen pharmazeutischen Zusammensetzung ausgewählt ist aus der Gruppe bestehend aus einer Tablette, einer Pille, einer Kapsel, einer Emulsion, einem Gel, einem Sirup und einer Suspension.

8. Pharmazeutische Zusammensetzung zur Verwendung nach einem der vorstehenden Ansprüche, **dadurch gekennzeichnet, dass** es sich bei dem *Scutellaria baicalensis* um eine oder mehrere Labiatae-Pflanzen handelt, ausgewählt aus der Gruppe bestehend aus *Scutellaria viscidula bunge, Scutellaria amoena, Scutellaria rehderiana Diels, Scutellaria ikonnikovii Juz, Scutellaria likiangensis* und *Scutellaria hypericifolia.*

9. Pharmazeutische Zusammensetzung zur Verwendung nach einem der vorstehenden Ansprüche, **dadurch gekennzeichnet, dass** die *Cortex Phellodendri* ausgewählt ist aus der Gruppe bestehend aus *Phellodendron chinense Schneid, Phellodendron amurense, Phellodendron chinense Schneid var. omeiense, Phellodendron Schneid var. yunnanense* und *Phellodendron chinense Schneid var. falcutum.*

10. Pharmazeutische Zusammensetzung zur Verwendung nach einem der vorstehenden Ansprüche, **dadurch gekennzeichnet, dass** der *Scutellaria baicalensis-Extrakt* ein in Sesamöl erhaltener *Scutellaria baicalensis-Extrakt* ist, der *Cortex Phellodendri-*Extrakt ein in Sesamöl erhaltener *Cortex Phellodendri-Extrakt* ist und der *Coptis chinensis-Extrakt* ein in Sesamöl erhaltener *Coptis chinensis-Extrakt* ist.

11. Pharmazeutische Zusammensetzung zur Verwendung nach einem der vorstehenden Ansprüche, **dadurch gekennzeichnet, dass** die pharmazeutische Zusammensetzung 7 % Bienenwachs, 1 % Sterol, 0,5 % Obaculacton, 0,3 % Baicalin und 0,5 % Berberin nach Gewicht auf der Grundlage des Gesamtgewichts der Zusammensetzung umfasst.

12. Pharmazeutische Zusammensetzung zur Verwendung nach einem der vorstehenden Ansprüche, **dadurch gekennzeichnet, dass** das Bienenwachs Mikrokristalle mit einer Länge von 0,1 bis 100 Mikrometern aufweist, wobei vorzugsweise mindestens zwei Mikrokristalle des Bienenwachses in der pharmazeutischen Zusammensetzung zu einem Mikrokristallkomplex polymerisiert sind, wobei bevorzugter die Mikrokristalle des Bienenwachses ausreichend gleichmäßig in dem genießbaren Öl dispergiert sind.

## Revendications

1. Composition pharmaceutique convenant à une administration orale destinée à être utilisée dans le traitement thérapeutique ou la prévention de la fibrose des organes, la formation de cicatrices et/ou le vieillissement des tissus, comprenant un mélange homogène d'huile comestible, de cire d'abeille, de β-sitostérol, de *Scutella baicalensis* ou d'un extrait de *Scutella baicalensis* contenant 0,1 à 0,5 % de baïcaline, de *Cortex Phellodendri* ou d'un extrait de *Cortex Phellodendri* contenant 0,1 à 1 % d'obaculactone, de *Coptis chinensis* ou d'un extrait de *Coptis chinensis* contenant 0,1 à 1 % de berbérine, de *Pericarpium Papaveris* ou d'un extrait de *Pericarpium Papaveris* contenant de 0,1 à 1 % de narcotoline, et de ver de terre ou d'un extrait de ver de terre contenant des acides aminés ; dans laquelle la cire d'abeille dans la composition forme des microcristaux, la teneur en cire d'abeille est de 0,5 à 50 % et la teneur en β-sitostérol est de 0,1 à 20 % en poids rapporté au poids de la composition, la composition pharmaceutique contient 2 à 5 % de *Scutella baicalensis* ou l'extrait de *Scutella baicalensis* contenant de 0,1 à 0,5 % de baïcaline, de 2 à 5 % de *Cortex Phellodendri* ou l'extrait de *Cortex Phellodendri* contenant de 0,1 à 1 % d'obaculactone, de 2 à 5 % de *Coptis chinensis* ou l'extrait de *Coptis chinensis* contenant de 0,1 à 1 % de berbérine, de 2 à 10 % de *Pericarpium Papaveris* ou l'extrait de *Pericarpium Papaveris* contenant de 0,1 de 1 % de narcotoline, et de 2 à 10 % de ver de terre ou d'extrait de ver de terre contenant des acides aminés, en poids rapporté au poids total de la composition, de préférence ledit organe comprend le cœur, le foie, les reins et la moelle des os, et provient d'un mammifère, de préférence un être humain.

2. Composition pharmaceutique destinée à être utilisée selon la revendication 1, **caractérisée en ce que** la teneur en β-sitostérol dans la composition pharmaceutique est de 0,5 à 20 % en poids, de préférence de 1 à 10 % en poids.

3. Composition pharmaceutique destinée à être utilisée selon la revendication 1, **caractérisée en ce que** la teneur en cire d'abeille dans la composition pharmaceutique est de 3 à 30 % en poids, de préférence de 5 à 20 % en poids, plus préférentiellement la teneur en cire d'abeille est de 6 à 10 % en poids

4. Composition pharmaceutique destinée à être utilisée selon l'une quelconque des revendications 1 à 3, **caractérisée en ce que** l'huile comestible dans la composition pharmaceutique est de l'huile de maïs, de l'huile de germe de blé, de l'huile de soja, de l'huile de son de riz, de l'huile de colza, de l'huile de sésame ou de l'huile de poisson.

5. Composition pharmaceutique destinée à être utilisée selon l'une quelconque des revendications précédentes, **caractérisée en ce que** la composition pharmaceutique comprend en outre de la propolis, et sa teneur est de 0,1 à 30 % en poids.

6. Composition pharmaceutique destinée à être utilisée selon l'une quelconque des revendications précédentes, **caractérisée en ce que** la composition pharmaceutique contient de l'eau, et sa teneur est inférieure ou égale à 1 % en poids.

7. Composition pharmaceutique destinée à être utilisée selon l'une quelconque des revendications précédentes, **caractérisée en ce que** la forme de dosage de la compoition pharmaceutique orale est choisie dans le groupe constitué d'un comprimé, d'une pilule, d'une capsule, d'un gel, d'un sirop et d'une suspension.

8. Composition pharmaceutique destinée à être utilisée selon l'une quelconque des revendications précédentes, **caractérisée en ce que** *Scutella baicalensis* est au moins une plante *Labiatae* choisie dans le groupe constituée de *Scutella viscidula bunge, Scutella amoena, Scutella rehderiana Diels, Scutella ikonnikovii Juz, Scutella likiangensis* et *Scutella hypericifolia.*

9. Composition pharmaceutique destinée à être utilisée selon l'une quelconque des revendications précédentes, **caractérisée en ce que** *Cortex Phellodendri* est choisi dans le groupe constitué de *Phellodendron chinense Schneid, Phellodendron amurene, Phellodendron chinense Schneid var. omeiense, Phellodendron Schneid var. yunnanense* et *Phellodendron chinense Schneid var. falcutum.*

10. Composition pharmaceutique destinée à être utilisée selon l'une quelconque des revendications précédentes, **caractérisée en ce que** l'extrait de *Scutella baicalensis* est un extrait de *Scutella baicalensis* obtenu dans de l'huile de sésame, l'extrait de *Cortex Phellodendri* est un extrait de *Cortex Phellodendri* obtenu dans de l'huile de sésame, et l'extrait de *Coptis chinensis* est un extrait de *Coptis chinensis* obtenu dans de l'huile de sésame.

11. Composition pharmaceutique destinée à être utilisée selon l'une quelconque des revendications précédentes, **caractérisée en ce que** la composition pharmaceutique contient 7 % de cire d'abeille, 1 % de stérol, 0,5 % d'obaculactone, 0,3 % de baïcaline et 0,5 % de berbérine en poids rapporté au poids de la composition.

12. Composition pharmaceutique destinée à être utilisée selon l'une quelconque des revendications précédentes, **caractérisée en ce que** la cire d'abeillle a des microcristaux avec une longueur de 0,1 à 100 microns, de préférence deux microcristaux de la cire d'abeille dans la composition pharmaceutique sont polymérisés en un complexe microcristallin, plus préférentiellement lesdits microcristaux de cire d'abeille sont assez uniformément dispersés dans l'huile comestible.
